# EUROPEAN PATENT SPECIFICATION

(11) **EP 2 518 479 B1**
(45) Date of publication and mention of the grant of the patent: **11.02.2015**
(21) Application number: 10839555.9
(22) Date of filing: 24.12.2010
(51) Int. Cl.: G01N 25/56, A61F 5/44, A61F 13/42

(54) **EXCRETION DETECTION DEVICE AND ABSORBABLE SUBSTANCE**
AUSSCHEIDUNGSERKENNUNGSVORRICHTUNG UND ABSORBIERBARE SUBSTANZ
DISPOSITIF DE DÉTECTION D'EXCRÉTION ET SUBSTANCE ABSORBABLE

(30) Priority: 24.12.2009 JP 2009293503
(43) Date of publication of application: 31.10.2012
(73) Proprietor: Unicharm Corporation, Ehime 799-0111 (JP)
(72) Inventor: SUZUKI, Miou, Kanonji-shi Kagawa 769-1602 (JP); FUJIOKA, Yoshihisa, Kanonji-shi Kagawa 769-1602 (JP)
(74) Representative: Eke, Philippa Dianne
(86) International application number: PCT/JP2010/073345
(87) International publication number: WO 2011/078325

(56) References cited:
- WO-A1-00/00150
- WO-A1-2008/026092
- JP-A- 2000 185 067
- JP-A- 2002 022 687
- JP-A- 2002 143 199
- JP-A- 2002 277 435
- JP-A- 2002 301 098
- JP-A- 2005 000 602
- JP-A- 2009 210 533
- US-A- 5 959 535
- US-A1- 2005 137 542

## Description

### [Technical Field]

The present invention relates to an excretion detection device, by which the excretion of bodily waste from a living body is detected, and an absorbent article including such an excretion detection device.

### [Background Art]

As regards the absorbent article such as a disposable diaper, a method of detecting the excretion of urine or stool from a wearer (living body) of the absorbent article is known.

For example, a method of electrification by bringing the bodily waste of the wearer in contact with a pair of electrodes configured from materials having different ionization tendencies, is known. That is, by making use of the principle of a voltaic cell, a method of operating an alarm such as a buzzer without using a battery has been proposed (see Patent Document 1).

On the other hand, a method of loading an excretion detection device making use of both electrification through the bodily waste (urine) and a battery, in a disposable diaper, has been proposed (see Patent Document 2). According to the excretion detection device, the consumption of the battery can be controlled, and the excretion from the wearer can be detected over a long period of time.

Furthermore, in order to more certainly detect the excretion from the wearer, a method of using a sensor for detecting environment information that changes as a result of excretion from the wearer, such as temperature, humidity, or odor has been proposed (see Patent Document 3).

### [Prior Art Document]

### [Patent Document]

[Patent Document 1]: Japanese Patent Application Publication No. 2002-277435 (Page 3, Fig. 4)
[Patent Document 2]: Japanese Patent Application Publication No. 4-138155 (Pages 2 to 3, Fig. 1)
[Patent Document 3]: Japanese Patent Application Publication No. 2006-296566 (Page 4, Fig. 1)

### [Summary of the Invention]

When using a sensor for detecting the temperature, for example, as described above, in order to more certainly detect the excretion from the wearer, it is desired to operate the sensor before the wearer excretes urine or stool, and to acquire environment information such as the temperature as needed. For example, when using a temperature sensor, in order to detect the rise in the temperature that occurs immediately after the excretion of urine or stool, it is necessary to compare the temperature before excretion and the temperature after excretion.

However, as described in Patent Document 1, when electrification is performed using bodily waste, electrification cannot be performed before the excretion of urine or stool, and therefore the sensor cannot be operated, which is a problem.

On the other hand, as described in Patent Document 2, when a battery is also used therewith, the sensor can be operated before excretion; however, as a result of loading a battery, other problems emerge in that a reduction in the size and weight of the excretion detection device and ease of disposability after use are hindered.

Therefore, an object of the present invention is to provide an excretion detection device by which a reduction in the size and weight, and ease of disposability after use can be achieved, while more certainly detecting the excretion of urine or stool from the wearer, and also to provide an absorbent article equipped with such an excretion detection device.

To solve the aforementioned problem, the present invention provides the excretion detection device of independent Claim 1 and the absorbent article of Claim 4. The remaining claims specify preferred but optional features.

A feature of the present invention is an excretion detection device (excretion detection device 100) configured to detect the excretion of bodily waste (urine or stool) from a living body (wearer W), comprising: a power supply unit (power supply unit 160) having a pair of electrodes (electrode 121, electrode 122) configured by using materials having different ionization tendencies; a solution retention unit (solution retention unit 110) configured to retain an electrolyte solution; a sensor (temperature sensor 130) operated by the electric power generated by the power supply unit, and configured to detect environment information (e.g., temperature information) that changes as a result of excretion from the living body; and a notification unit (radio transmission unit 180) operated by the electric power generated by the power supply unit, and configured to notify the environment information detected by the sensor to outside the excretion detection device, wherein the pair of electrodes are installed at a position where the electrodes can be in contact with at least the electrolyte solution.

Another feature of the present invention is an absorbent article (e.g., disposable diaper 10A) worn by a living body and configured to absorb bodily waste from the living body, wherein the absorbent article includes an excretion detection device configured to detect the excretion of the bodily waste, and the excretion detection device comprises: a power supply unit having a pair of electrodes configured by using materials having different ionization tendencies; a solution retention unit configured to retain an electrolyte solution; a sensor operated by the electric power generated by the power supply unit, and configured to detect environment information that changes as a result of excretion from the living body; and a notification unit operated by the electric power generated by the power supply unit, and configured to notify the environment information detected by the sensor to outside the excretion detection device, wherein the pair of electrodes are installed at a position where the electrodes can be in contact with at least the electrolyte solution.

### [Brief Description of the Drawings]

[Fig. 1] Fig. 1 is a diagram showing an entire schematic configuration of an excretion management system 1 according to an embodiment of the present invention.
[Fig. 2] Fig. 2 is a developed plan view of a disposable diaper 10A in which an excretion detection device 100 according to an embodiment of the present invention is embedded, and is also a schematic illustration of the appearance of the excretion detection device 100.
[Fig. 3] Fig. 3 is a diagram showing a functional block configuration of the excretion detection device 100 according to the embodiment of the present invention.
[Fig. 4] Fig. 4 is a diagram showing the excretion management flow using the excretion management system 1 according to the embodiment of the present invention.
[Fig. 5] Fig. 5 is a graph showing a temperature change pattern during defecation, and a temperature change pattern during urination.

### [Description of Embodiments]

Next, embodiments of an excretion detection device and an absorbent article according to the present invention are explained with reference to the drawings. In the following description of the drawings, the same or similar reference numerals are used to designate the same or similar parts. It will be appreciated that the drawings are schematically shown and the ratio and the like of each dimension are different from the real ones.

Therefore, a specific dimension should be determined in view of the following description. Moreover, among the drawings, the respective dimensional relations or ratios may differ.

### (1) Entire schematic configuration of an excretion management system

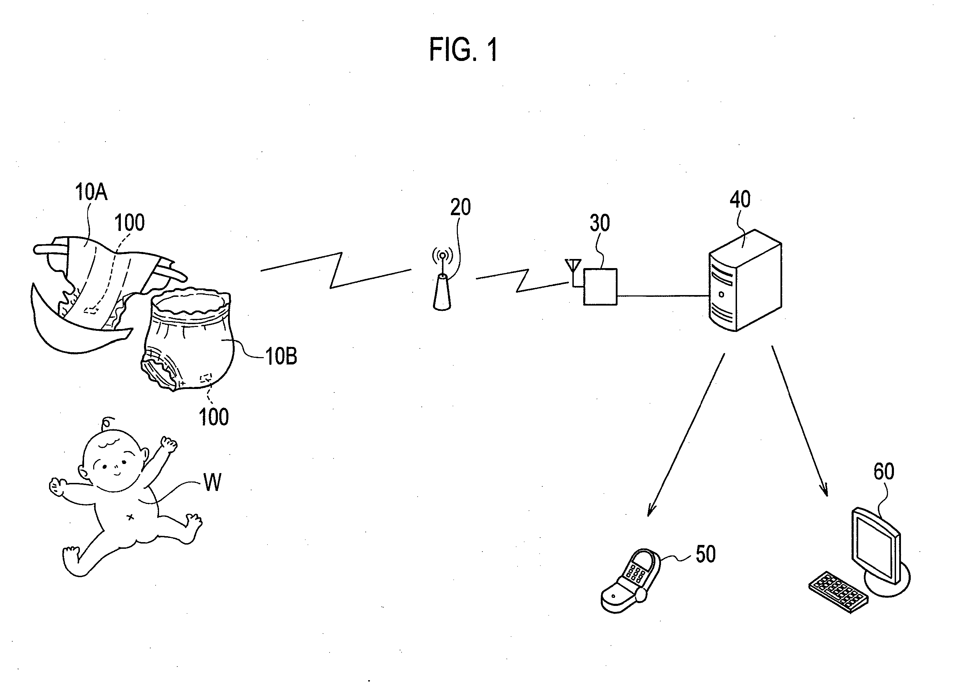

Fig. 1 is a diagram showing an entire schematic configuration of an excretion management system 1 according to the present embodiment. As shown in Fig. 1, the excretion management system 1 is configured from a radio relay station 20, an excretion management server 40, and an excretion detection device 100.

A disposable diaper 10A and a disposable diaper 10B are absorbent articles worn by a wearer W (living body), and absorbing a bodily waste such as urine or stool from the wearer W. The disposable diaper 10A and the disposable diaper 10B are equipped with the excretion detection device 100 configured to detect the excretion of bodily waste. Specifically, the excretion detection device 100 is installed in the open-type (tape-type) disposable diaper 10A and the pant-type disposable diaper 10B.

The excretion detection device 100 detects the excretion of bodily waste from the wearer W. The excretion detection device 100 is configured from an active-type IC tag that can transmit a radio signal to the radio relay station 20, and a temperature sensor, and repetitively transmits radio signals including the temperature information (environment information) within the disposable diaper, at a predetermined period. The detailed configuration of the excretion detection device 100 is described later.

The radio relay station 20 converts the radio signal received from the excretion detection device 100 to a radio signal in accordance with a predetermined communication system. In the present embodiment, the radio relay station 20 converts the radio signal received from the excretion detection device 100 to a radio signal of a Personal Handy-phone (PHS system), and then transmits the radio signal to a radio base station 30. The radio relay station 20 is a rectangular parallelepiped with each side of a few centimeters or less, which can be installed as a name tag in the bed, and can be installed on the wearer W. In order to maintain radio communication between the radio relay station 20 and the excretion detection device 100, the radio relay station 20 and the excretion detection device 100 must be positioned within a fixed distance.

The radio base station 30 is configured to receive a radio signal from the radio relay station 20. In the present embodiment, the radio base station 30 transmits and receives radio signals in accordance with the PHS system. The radio base station 30 is connected to the excretion management server 40 via a wire communication network (not shown).

The excretion management server 40 stores a plurality of change patterns of the environment information (such as the temperature, humidity, and odor) concerning defecation and urination. The excretion management server 40 determines the existence or non-existence of urination or defecation by the wearer W based on the temperature information transmitted repetitively from the excretion detection device 100 via the radio relay station 20 and the radio base station 30. Upon determining the occurrence of urination or defecation, the excretion management server 40 transmits an email indicating the same to a pre-registered cellular phone terminal 50 or a personal computer 60. When the excretion management server 40 transmits such an email to a pre-registered address in the cellular phone terminal 50 or the personal computer 60, a guardian (carer) of the wearer W, who checks the email can quickly take an appropriate measure, such as changing the disposable diaper.

### (2) Layout position and structure of the excretion detection device

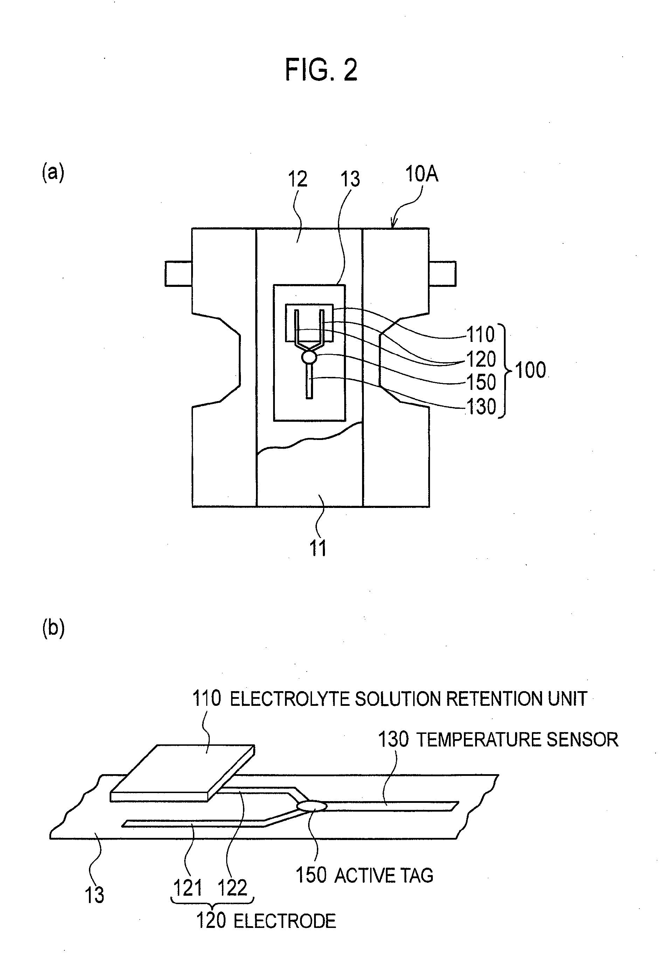

Fig. 2 (a) is a developed plan view of the disposable diaper 10A in which the excretion detection device 100 is embedded. Fig. 2 (b) is a schematic illustration of the appearance of the excretion detection device 100.

As shown in Fig. 2 (a), the disposable diaper 10A includes a topsheet 11, a backsheet 12, and an absorber 13. The topsheet 11 is in contact with the skin of the wearer W (living body). The topsheet 11 is configured from a material that allows liquids such as urine to pass through. The backsheet 12 is in contact with the clothing of the wearer W. The backsheet 12 is configured from a material that does not allow liquids to pass through. The absorber 13 absorbs bodily waste, such as urine or stool. The absorber 13 is configured from hydrophilic fibers such as ground pulp. The disposable diaper 10A can be manufactured according to a known method (for example, Japanese Patent Application Publication No. JP2003-339771).

The excretion detection device 100 is provided between the topsheet 11 and the absorber 13. The excretion detection device 100 may be provided between the backsheet 12 and the absorber 13; however, in order to be sure that the stool, which has a higher viscosity as compared to urine, is brought in contact with an electrode unit 120 of the excretion detection device 100, it is desired that the excretion detection device 100 be provided between the topsheet 11 and the absorber 13. Furthermore, even in the case of a pants-type disposable diaper 10B, the excretion detection device 100 is desired to be provided between the topsheet 11 and the absorber 13. The disposable diaper 10B can be also manufactured according to a known method (for example, Japanese Patent Application Publication No. JP2005-58755).

The excretion detection device 100 includes a solution retention unit 110, an electrode unit 120, a temperature sensor 130, and an active tag 150. The solution retention unit 110 retains an electrolyte solution. The electrode unit 120 is provided at the lower side of the solution retention unit 110 (towards the absorber 13). In order to prevent it coming in contact with the electrolyte solution seeping out from the electrolyte retention unit, the temperature sensor 130 is provided on the opposite side of an electrode 121 and an electrode 122, with reference to the active tag 150. The electrode 121 and the electrode 122 are provided at a position where the electrodes 121 and 122 can be in contact with the bodily waste from the wearer W and the electrolyte solution seeping out from the solution retention unit 110. That is, the electrode 121 and the electrode 122 can be brought in contact with the electrolyte solution seeping out from the solution retention unit 110 before the excretion of urine, for example.

The active tag 150 is connected to the electrode unit 120 and the temperature sensor 130, and is provided at a position where the active tag 150 does not overlap the solution retention unit 110.

### (3) Functional block configuration of the excretion detection device 100

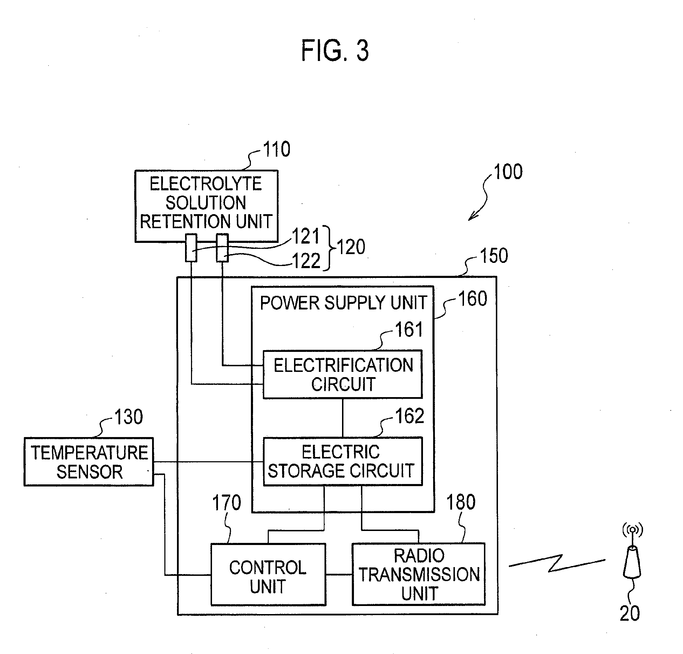

Fig. 3 is a diagram illustrating the functional block configuration of the excretion detection device 100. As shown in Fig. 3, the excretion detection device 100 includes the solution retention unit 110, the electrode unit 120, the temperature sensor 130, and the active tag 150. Furthermore, the active tag 150 is configured from a power supply unit 160, a control unit 170, and a radio transmission unit 180.

The solution retention unit 110 retains an electrolyte solution prepared by dissolving an electrolyte in a solvent such as water. For example, the solution retention unit 110 can use an aqueous solution including approx. 1 wt. % to 5 wt. % of sodium chloride (NaCl). That is, a NaCl aqueous solution similar to the urinary constituent is used as the electrolyte solution. The solution retention unit 110 is a container having the shape of a bag that can store the liquids, and in the present embodiment, the solution retention unit 110 is configured from a thermoplastic film of polyolefin.

By performing a predetermined operation for the solution retention unit 110, for example, by pressing the solution retention unit 110 from the topsheet 11 side, the electrolyte solution can be made to seep out from the solution retention unit 110. That is, by pressing the solution retention unit 110, the bag-shaped solution retention unit 110 configured from a thermoplastic film is torn, and the electrolyte solution seeps out near the electrode unit 120. As a result, the electrolyte solution comes in contact with the electrode 121 and the electrode 122.

The solution retention unit 110 may store only solvents such as water while powdered sodium chloride may be placed in the proximity of the solution retention unit 110. In such a case, the powdered sodium chloride dissolves in the water seeping out from the solution retention unit 110 to result in an electrolyte solution. Even in such a case, the solution retention unit 110 is said to retain an electrolyte solution.

The electrode unit 120 is provided at the lower side of the solution retention unit 110 (towards the absorber 13), and above the absorber 13. The pair of electrode 121 and electrode 122 configuring the electrode unit 120 are connected to the power supply unit 160. The electrode 121 and the electrode 122 are configured using materials having different ionization tendencies. In the present embodiment, a combination of aluminum and silver is used as the electrode 121 and the electrode 122. That is, the power supply unit 160 having such an electrode unit 120 functions as a so-called voltaic cell through the interposition of the electrolyte solution or the bodily waste between the electrode 121 and the electrode 122. A voltaic cell is a cell that generates electric current by moving electrons through the immersion of electrodes using a metal with a large ionization tendency and a metal with a small ionization tendency, in an electrolyte solution.

As described above, the electrode 121 and the electrode 122 can be brought in contact with the electrolyte solution seeping out from the solution retention unit 110 before the excretion of urine, for example. Therefore, a difference in potential occurs between the electrode 121 and the electrode 122 due to the electrolyte solution prior to excretion, and due to the electrolyte solution or the bodily waste after excretion, and electromotive force is obtained.

The temperature sensor 130 is configured to detect the environment information that changes as a result of excretion from the wearer W. Specifically, in the disposable diaper 10A, the temperature sensor 130 detects the temperature of the region where the bodily waste is excreted (the region in which the absorber 13 is provided) as the environment information. The temperature sensor 130 is operated by the electric power generated by the power supply unit 160.

In the present embodiment, a thin-film thermistor (hereinafter, thermistor) is used as the temperature sensor 130. In the temperature sensor 130, a synthetic-resin film is used on which the electrode for detecting the urine or stool, and the electrode for supplying electric power to the thermistor are printed with a conductive ink. A thermistor having a small heat capacity and easily affected by the surrounding temperature, for example, the ET-103 thermistor manufactured by SEMITEC CORPORATION, is desired to be used as the temperature sensor 130.

The active tag 150 is an active-type IC tag with which the electrode unit 120 and the temperature sensor 130 are connected. That is, the active tag 150 can transmit a radio signal without depending on another radio communication device, for example.

The power supply unit 160 has an electrification circuit 161 and an electric storage circuit 162. The electrode 121 and the electrode 122 are connected to the electrification circuit 161. The electrification circuit 161 is electrified due to the difference in potential that occurs between the electrode 121 and the electrode 122, and stores the generated electric power in the electric storage circuit 162. The electric storage circuit 162 is configured from a capacitor, for example.

The control unit 170 is operated by the electric power generated by the power supply unit 160. Specifically, based on the value output from the temperature sensor 130, the control unit 170 generates data indicating the temperature information, and then outputs the generated data to the radio transmission unit 180.

The radio transmission unit 180 is operated by the electric power generated by the power supply unit 160. The radio transmission unit 180 notifies the temperature information detected by the temperature sensor 130 to outside the excretion detection device 100. In the present embodiment, the radio transmission unit 180 configures a notification unit.

Specifically, the radio transmission unit 180 transmits a radio signal including the data indicating the temperature information towards the radio relay station 20. In the present embodiment, the radio transmission unit 180 repetitively transmits radio signals at a predetermined period (for example, 10 seconds) .

As described above, because the excretion detection device 100 functions as a voltaic cell before and after excretion, the excretion detection device is a battery-less type device in which a battery is not loaded for operating the temperature sensor 130, the control unit 170, and the radio transmission unit 180.

### (4) Excretion management method using the excretion management system

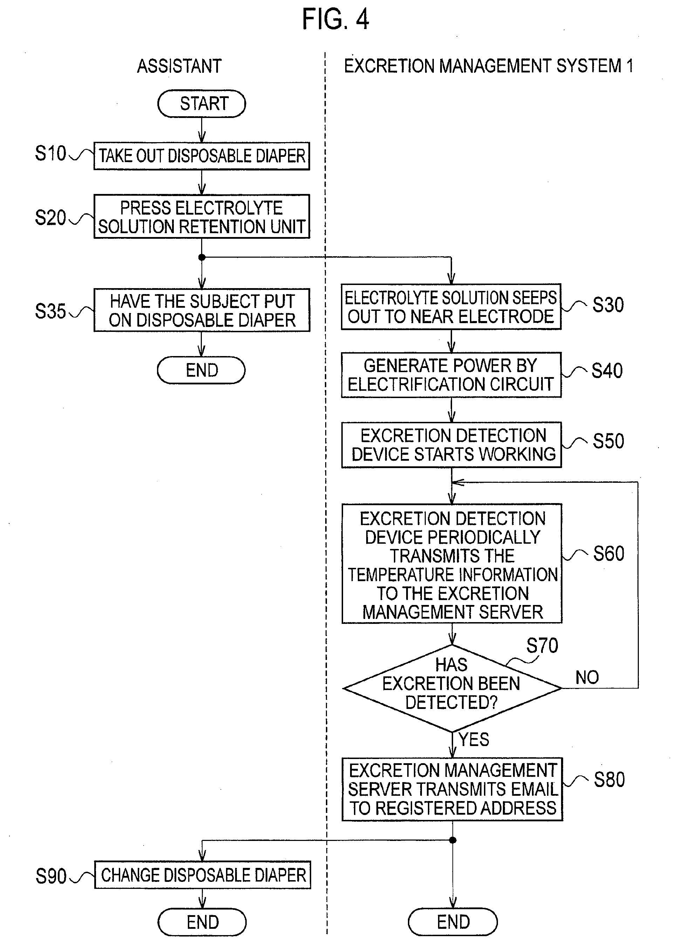

Fig. 4 shows the excretion management flow using the aforementioned excretion management system 1. Specifically, Fig. 4 shows the flow from the detection of urination or defecation, by the excretion detection device 100, from the wearer W wearing the disposable diaper 10A (or the disposable diaper 10B), up to the transmission of an email notifying the same to the cellular phone terminal 50 or the personal computer 60.

As shown in Fig. 4, in step S10, a guardian or a carer (hereinafter, assistant) assisting subjects such as infants and care-receivers in wearing the disposable diaper 10A, and people requiring a carer in wearing the disposable diaper 1 takes out the disposable diaper 10A from the package.

In step S20, the assistant presses the solution retention unit 110 embedded at the lower side of the topsheet 11 of the disposable diaper 10A. As a result of such an operation by the assistant, the bag-shaped solution retention unit 110 is torn.

In step S30, the electrolyte solution (sodium chloride solution) seeps out from the solution retention unit 110 near the electrode unit 120.

In step S35, the assistant has the subject, such as an infant or toddler, put on the disposable diaper 10A. The task of putting on the disposable diaper 10A by the assistant is thus complete.

In step S40, the power supply unit 160 (electrification circuit 161) is electrified due to the interposition of the electrolyte solution between the electrode 121 and the electrode 122, and generates electric power.

In step S50, the excretion detection device 100 starts operating as a result of the electric power generated by the power supply unit 160.

In step S60, the excretion detection device 100 periodically transmits the temperature information acquired by the temperature sensor 130 to the excretion management server 40. Specifically, as described above, the excretion detection device 100 transmits the temperature information to the excretion management server 40 via the radio relay station 20 and the radio base station 30.

In step S70, based on the received temperature information, the excretion management server 40 determines whether or not the temperature change pattern matches the already-stored pattern. In the present embodiment, the excretion management server 40 stores the temperature change pattern that occurs as a result of urination, and the temperature change pattern that occurs as a result of defecation.

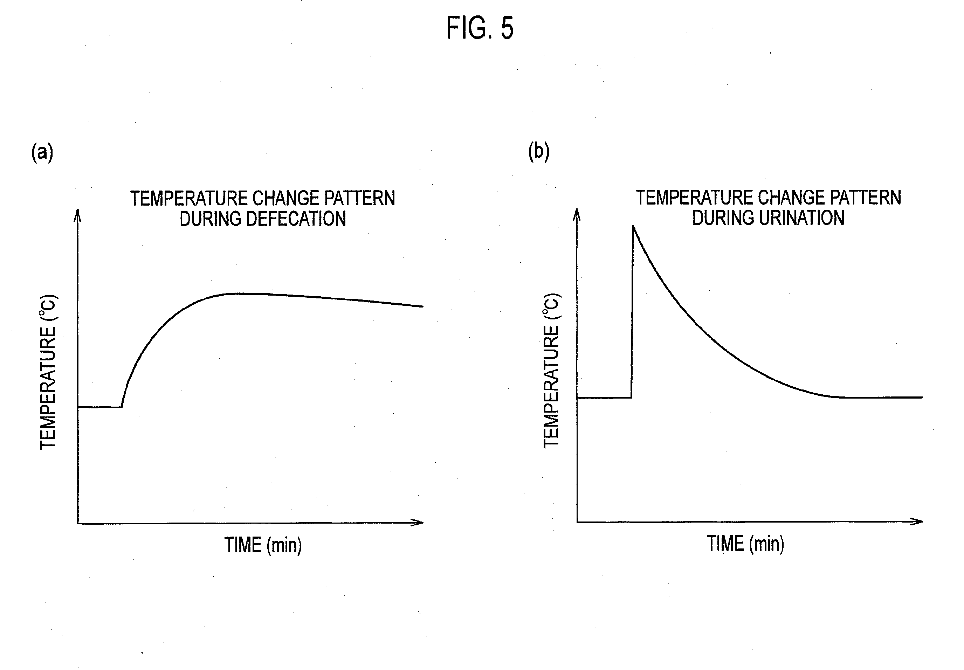

Fig. 5 (a) shows the temperature change pattern that occurs during defecation. Fig. 5 (b) shows the temperature change pattern that occurs during urination. When the temperature change pattern during defecation is compared with the temperature change pattern during urination, the rise in the temperature is sharp during urination as compared to defecation, and the convergence of temperature change is also fast. On the other hand, the rise in the temperature is slow during defecation, and the convergence of temperature change is also slow. Based on such differences in the temperature change pattern, the excretion management server 40 identifies urination or defecation.

When the temperature change pattern based on the received temperature information matches the temperature change pattern of urination or defecation (YES in step S70), then in step S80, the excretion management server 40 transmits an email indicating the occurrence of urination or defecation to a pre-registered address (cellular phone terminal 50 or personal computer 60).

In step 90, as a result of receipt of an email, the assistant of the wearer W quickly recognizes urination or defecation by the wearer W, and changes the disposable diaper. In order to prevent interference between the radio signal transmitted by the excretion detection device 100 embedded in the disposable diaper after use, and the radio signal transmitted by the excretion detection device 100 embedded in the new disposable diaper after change, the assistant must take away the disposable diaper after use by more than a fixed distance from the radio relay station 20.

According to the aforementioned excretion detection device 100, the electrode unit 120 is provided at a position where the electrode unit 120 can be in contact with the bodily waste of the wearer W and the electrolyte solution retained by the solution retention unit 110. Additionally, the electrode unit 120 can also be in contact with the electrolyte solution before excretion by the wearer W.

Therefore, electrification by the power supply unit 160 is possible even before urination or defecation, and the temperature sensor 130 can be operated. Furthermore, because such a configuration is adopted in the excretion detection device 100, a battery is not loaded at all. That is, according to the excretion management system 1 including the excretion detection device 100, by continuously acquiring the temperature information using the temperature sensor 130, the excretion of urine or stool from the wearer W can be more certainly detected. Furthermore, because no battery is loaded, a reduction in the size and weight of the excretion detection device 100, and ease of disposability after use can be achieved.

Additionally, according to the excretion management system 1, by receiving an email, the guardian or the carer assisting in wearing the disposable diaper 10A can quickly recognize the excretion of urine or stool by the wearer W, and can take an appropriate measure, such as changing the disposable diaper.

Furthermore, according to the excretion detection device 100, because the electrode unit 120 is provided at a position where the electrode unit 120 can be in contact with the bodily waste of the wearer W, even when electrification by the electrolyte solution seeping out from the solution retention unit 110 can no longer be performed, electrification can be continued by using the bodily waste, and the temperature information can be transmitted over a long period of time.

### (5) Other embodiments

As described above, the present invention is disclosed through the above embodiments. However, it should not be interpreted that the statements and drawings constituting a part of the present disclosure limit the present invention. From this disclosure, a variety of alternate embodiments, examples, and applicable techniques will become apparent to one skilled in the art.

In the aforementioned embodiment, the solution retention unit 110 was torn and the electrolyte solution was made to seep out from the solution retention unit 110 by pressing the solution retention unit 110 from the topsheet 11 side. However, the electrolyte solution may be made to seep out by performing an operation other than pressing. For example, the solution retention unit 110 can be torn by pulling the thread-like members connected to the solution retention unit 110.

In the aforementioned embodiment, the excretion detection device 100 was embedded in the disposable diaper. However, the excretion detection device 100 may be loaded in a diaper that can be used repetitively several times. Furthermore, the excretion detection device 100 is not just loaded in a diaper, but may also be loaded in an absorbent article such as a sanitary napkin. Additionally, the absorbent article is not just used for human beings, but may also be used for animals, such as pets.

In the aforementioned embodiment, the excretion detection device 100 was a battery-less type device. However, a battery may be loaded as long as the loaded battery is small in size and is lightweight, and the ease of disposability is provided.

As described above, needless to say, the present invention includes various embodiments and the like not described here. Therefore, the scope of the present invention is to be defined only by the inventive specific matter according to the adequate claims from the above description.

According to the present invention, it is possible to provide an excretion detection device by which a reduction in the size and weight, and the ease of disposability after use can be achieved, while more certainly detecting the excretion of urine or stool from the wearer, and also to provide an absorbent article equipped with such an excretion detection device.

### [Explanation of the Reference Signs]

1 ... Excretion management system, 10A, 10B ... Disposable diaper, 11 ... Topsheet, 12 ... Backsheet, 13 ... Absorber, 20 ... Radio relay station, 30 ... Radio base station, 40 ... Excretion management server, 50 ... Cellular phone terminal, 60 ... Personal computer, 100 ... Excretion detection device, 110 ... Solution retention unit, 120 ... Electrode unit, 121, 122 ... Electrode, 130 ... Temperature sensor, 150 ... Active tag, 160 ... Power supply unit, 161 ... Electrification circuit, 162 ... Electric storage circuit, 170 ... Control unit, 180 ... Radio transmission unit, W ... Wearer

## Claims

1. An excretion detection device (100) for detecting the excretion of bodily waste from a living body, comprising:
a power supply unit (160) having a pair of electrodes (121, 122) for which materials having different ionization tendencies are used;
a solution retention unit (110) retaining an electrolyte solution;
a sensor (130) operated by the electric power generated by the power supply unit, and adapted to detect environment information that changes as a result of excretion from the living body; and
a notification unit (180) operated by the electric power generated by the power supply unit, and adapted to notify the environment information detected by the sensor to outside the excretion detection device, wherein
the pair of electrodes are installed at a position where the electrodes are able to come into contact with the bodily waste and the electrolyte solution, and are also able to come into contact with the electrolyte solution before the excretion of the bodily waste;
the solution retention unit (110) causes the electrolyte solution to seep out from the solution retention unit by the solution retention unit being torn, and the electrolyte solution is brought in contact with the pair of electrodes;
the notification unit (180) is a radio transmission unit adapted to transmit a radio signal including the data indicating the environment information detected by the sensor;
the excretion detection device is installed in an absorbent article for wearing by the living body; and
in the absorbent article, the sensor detects the temperature of the region in which the bodily waste is excreted as the environment information.

2. An absorbent article (10A, 10B) for wearing by a living body and adapted to absorb bodily waste from the living body, wherein
the absorbent article includes an excretion detection device (100) as claimed in claim 1.

3. The absorbent article according to claim 2, wherein
the absorbent article comprises:
a topsheet (11) that is adapted to be in contact with the living body and allows the passage of liquids; and
an absorber (13) adapted to absorb the bodily waste, wherein
the excretion detection device (100) is provided between the topsheet and the absorber.

## Patentansprüche

1. Vorrichtung zur Ausscheidungserkennung (100) zum Erkennen der Ausscheidung von Fäkalien von einem lebenden Körper, die Folgendes umfasst:
ein Netzgerät (160), das ein Elektrodenpaar (121, 122) hat, für das Materialien mit unterschiedlichen lonisierungstendenzen verwendet werden,
eine Aufbewahrungseinheit (110) für eine Lösung, die eine Elektrolytlösung enthält,
einen Sensor (130), der von einer elektrischen Energie betrieben wird, die vom Netzgerät generiert wird und so adaptiert ist, dass es Umweltinformationen entdeckt, die sich als Ergebnis der Ausscheidung von einem lebenden Körper ändern, und
eine Anzeigeeinheit (180), die von der elektrischen Energie betrieben wird, die vom Netzgerät generiert wird und so adaptiert ist, dass es die Umweltinformationen meldet, die vom Sensor der Vorrichtung zur Ausscheidungerkennung erkannt werden, wobei
das Elektrodenpaar an einer Position installiert ist, wobei die Elektroden in der Lage sind, mit den Fäkalien und der Elektrolytlösung in Berührung zu kommen, und auch in der Lage ist, mit der Elektrolytlösung vor der Ausscheidung der Fäkalien in Berührung zu kommen,
die Aufbewahrungseinheit (110) für eine Lösung, die verursacht, dass die Elektrolytlösung von der Aufbewahrungseinheit heraussickert, wenn die Aufbewahrungseinheit für eine Lösung eingerissen ist, und die Elektrolytlösung mit dem Elektrodenpaar in Berührung gebracht wird,
die Anzeigeeinheit (180) eine Funkübertragungseinheit ist, die so adaptiert ist, dass sie ein Funksignal überträgt, einschließlich der Daten, die die Umweltinforationen anzeigen, die vom Sensor entdeckt werden,
die Vorrichtung zur Ausscheidungserkennung in einem saugfähigen Artikel zum Tragen von lebenden Körper installiert wird, und
im saugfähigen Artikel der Sensor die Temperatur des Bereichs, in dem die Fäkalien ausgeschieden werden, als Umweltinformationen entdeckt.

2. Saugfähiger Artikel (10A, 10B) zum Tragen eines lebenden Körpers, der so adaptiert ist, dass er Fäkalien vom lebenen Körper absorbiert, wobei
der saugfähige Artikel eine Vorrichtung zur Ausscheidungserkennung (100) beinhaltet, wie nach Anspruch 1.

3. Saugfähiger Artikel nach Anspruch 2, wobei der saugfähige Artikel Folgendes umfasst:
eine oberste Schicht (11), die so adaptiert ist, dass sie mit dem lebenden Körper in Berührung kommt und den Durchgang von Flüssigkeiten ermöglicht, und
einen Absorber (13), der so adaptiert ist, dass er die Fäkalien absorbiert, wobei
die Vorrichtung zur Ausscheidungserkennung (100) zwischen der obersten Schicht und dem Absorber bereitgestellt wird.

## Revendications

1. Dispositif de détection d'excrétion (100) permettant de détecter l'excrétion de déchets organiques en provenance d'un corps vivant, comportant :
un bloc d'alimentation (160) ayant des électrodes groupées par paire (121, 122) pour lesquelles des matériaux ayant différentes tendances d'ionisation sont utilisés ;
une unité de retenue de solution (110) permettant de retenir une solution électrolytique ;
un capteur (130) actionné par la puissance électrique générée par le bloc d'alimentation, et adapté pour détecter des informations se rapportant à l'environnement qui changent en fonction de l'excrétion en provenance du corps vivant ; et
une unité de notification (180) actionnée par la puissance électrique générée par le bloc d'alimentation, et adaptée pour transmettre les informations se rapportant à l'environnement détectées par le capteur à l'extérieur du dispositif de détection d'excrétion, dans lequel
les électrodes groupées par paire sont installées au niveau d'une position où les électrodes sont en mesure d'entrer en contact avec les déchets organiques et la solution électrolytique, et sont également en mesure d'entrer en contact avec la solution électrolytique avant l'excrétion des déchets organiques ;
l'unité de retenue de solution (110) amène la solution électrolytique à suinter hors de l'unité de retenue de solution par le déchirement de l'unité de retenue de solution, et la solution électrolytique est mise en contact avec les électrodes groupées par paire ;
l'unité de notification (180) est une unité de transmission radioélectrique adaptée pour transmettre un signal radioélectrique comprenant les données indiquant les informations se rapportant à l'environnement détectées par le capteur ;
le dispositif de détection d'excrétion est installé dans un article absorbant destiné à être porté par le corps vivant ; et
dans l'article absorbant, le capteur détecte la température de la région dans laquelle les déchets organiques sont excrétés comme informations se rapportant à l'environnement.

2. Article absorbant (10A, 10B) destiné à être porté par un corps vivant et adapté pour absorber des déchets organiques en provenance du corps vivant, dans lequel
l'article absorbant comprend un dispositif de détection d'excrétion (100) selon la revendication 1.

3. Article absorbant selon la revendication 2, dans lequel l'article absorbant comporte :
une feuille de dessus (11) qui est adaptée pour être en contact avec le corps vivant et qui permet le passage de liquides ; et
un élément absorbant (13) adapté pour absorber les déchets organiques, dans lequel
le dispositif de détection d'excrétion (100) est mis en oeuvre entre la feuille de dessus et l'élément absorbant.
